# EUROPÄISCHE PATENTSCHRIFT

(11) **EP 0 937 441 B1**
(45) Veröffentlichungstag und Bekanntmachung des Hinweises auf die Patenterteilung: **01.09.2004**
(21) Anmeldenummer: 99107442.8
(22) Anmeldetag: 26.04.1994
(51) Int. Cl.: A61C 19/00, A61B 19/00

(54) **Vorrichtung zur Intensivreinigung von ärztlichen, insbesondere zahnärztlichen, Gegenständen**
Device for intensive cleaning of medical, especially dental articles
Dispositif pour le nettoyage intensif d'articles médicaux notamment dentaires

(30) Priorität: 15.07.1993 DE 4323816
(43) Veröffentlichungstag der Anmeldung: 25.08.1999
(62) Teilanmeldung aus: 94106516.1
(73) Patentinhaber: Sirona Dental Systems GmbH, 64625 Bensheim (DE)
(72) Erfinder: Hruza, David Dipl. Ing., 88339 Bad Waldsee (DE); Stetter-Alle, Raimund Dipl. Ing. (FH), 89073 Ulm (DE); Trackl, Karl, 89129 Langenau (DE); Sutter, Ralf Dipl. Ing (FH), 69469 Weinheim (DE); Beerstecher, Lutz Dipl. Ing., 64625 Bensheim (DE)
(74) Vertreter: Sommer, Peter

(56) Entgegenhaltungen:
- EP-A- 0 300 945
- DE-A- 3 018 872
- DE-A- 3 117 264
- DE-C- 561 205
- US-A- 4 552 728

## Beschreibung

Die Erfindung bezieht sich auf eine Vorrichtung zur Intensivreinigung von ärztlichen, insbesondere zahnärztlichen, Instrumenten sowie gegebenenfalls in deren Anwendungsbereich fallenden anderen Gegenständen.

Im medizinischen, insbesondere zahnmedizinischen, Anwendungsbereich müssen die dort eingesetzten Instrumente nach einer Patientenbehandlung einer Intensivreinigung unterzogen werden. Im zahnärztlichen Bereich sind dies Bohr- und Schleifinstrumente (Winkelstücke und Turbinenhandstücke), Spritzhandstücke sowie diverse andere Handinstrumente. Darüber hinaus sind häufig, und dies gilt vornehmlich für den Laborbereich, auch andere Gegenstände, wie Zahnbrücken, Zahnspangen, Gebisse od.dgl. einer solchen Intensivreinigung zu unterziehen.

Aus der DE-A1-30 18 872 ist ein Verfahren zum Reinigen von Gegenständen, insbesondere medizinischen Geräten, bekannt, bei dem Reinigungsflüssigkeit intermittierend auf die Gegenstände gespritzt wird, wobei die Pausen zwischen den Spritzvorgängen so lang sind, daß auf den Gegenständen gebildeter Film der Reinigungsflüssigkeit abgerissen ist, bevor ein weiterer Spritzvorgang beginnt. Das Einspritzen der Reinigungsflüssigkeit erfolgt bei dem bekannten Verfahren mittels Druckluft. Die nach diesem Verfahren arbeitende Vorrichtung ist nach Art einer Geschirrspülmaschine aufgebaut, d.h. die Gegenstände werden einerseits von oben über einen mit Spritzdüsen besetzten Dreharm und andererseits von unten über sog. Langdüsen, die sich in das Innere der zu reinigenden Gegenstände erstrecken, besprüht.

Aus der DE-A1-39 16 446 ist es zu allgemeinen Reinigungszwecken, insbesondere zur Reinigung von Gestein, Bauwerken od.dgl. bekannt, dem Druckwasserstrahl durch Injektorwirkung Luft kontinuierlich oder periodisch regelbar zuzusetzen. Hierzu ist der Druckwasserdüse am Druckwasseraustritt eine Mischkammer mit diversen Lufteintrittsöffnungen vorgelagert, über die Luft angesaugt wird.

In der DE-A1-31 17 264 ist eine Kassette zur Ablage und Aufbewahrung von zahnärztlichen Instrumenten beschrieben, welche zwei im wesentlichen waagerecht angeordnete, zylindrische Ständer enthält, die hintereinander mehrere Aufnahmeeinrichtungen für die Instrumente aufweisen. Die Ständer sind schwenkbar gelagert, so daß die aufgesteckten Instrumente einerseits zur Entnahme dargereicht, andererseits zur Ablage und Aufbewahrung vor, während und nach einer Sterilisation benutzt werden können.

Der im Anspruch 1 angegebenen Erfindung liegt die Aufgabe zugrunde, eine Vorrichtung anzugeben, welche zur Intensivreinigung von ärztlichen, insbesondere zahnärztlichen Gegenständen gut geeignet ist.

Die erfindungsgemäße Vorrichtung umfaßt eine die Gegenstände aufnehmende Kassette, die in eine druckdicht verschließbare Kammer einlegbar ist, in der der Reinigungsprozeß abläuft. Die Kassette enthält Adapter zur Halterung der Gegenstände. Um die gehalterten Gegenstände herum ist eine Vielzahl von Düsen angeordnet, die gezielt auf die Gegenstände gerichtet sind. Über die Düsen wird Reinigungsflüssigkeit auf die Außenflächen der Gegenstände geleitet.

Die erfindungsgemäße Vorrichtung wird nachfolgend anhand der Zeichnung näher erläutert.

Es zeigen:
- Figur 1: eine Ausführungsform der erfindungsgemäßen Vorrichtung in einer schaubildlichen Darstellung,
- Figur 2: eine Ausführungsform einer in die Vorrichtung nach Figur 1 einsetzbaren Instrumentenkassette in schaubildlicher Explosionsdarstellung,
- Figuren: 3 und 4 eine Ausführungsform einer Abdeckung an der Rückseite der in Figur 2 gezeigten Kassette,
- Figur 5: einen der in Figur 2 gezeigten Adapter und den rückwärtigen Teil eines dazu passenden Instruments im Längsschnitt,
- Figur 6: eine vereinfachte Darstellung der Anordnung einer mit Instrument bestückten Kassette im Gerät,
- Figur 7: ein hydraulisch/pneumatisches Schaltbild,
- Figur 8: ein Impuls-Zeitdiagramm,
- Figur 9: eine Variante zu der in Figur 6 gezeigten Ausführungsform.

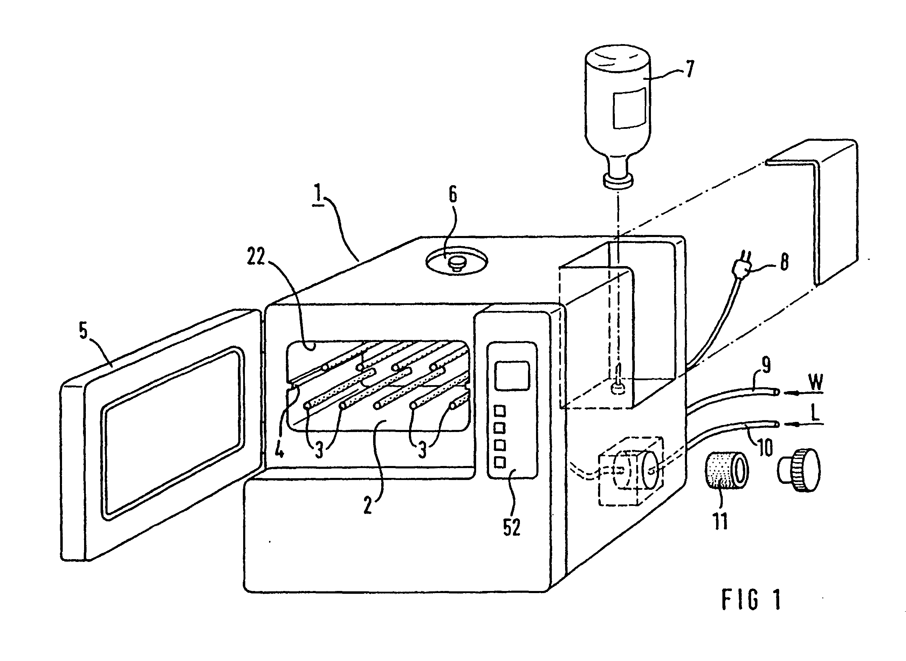

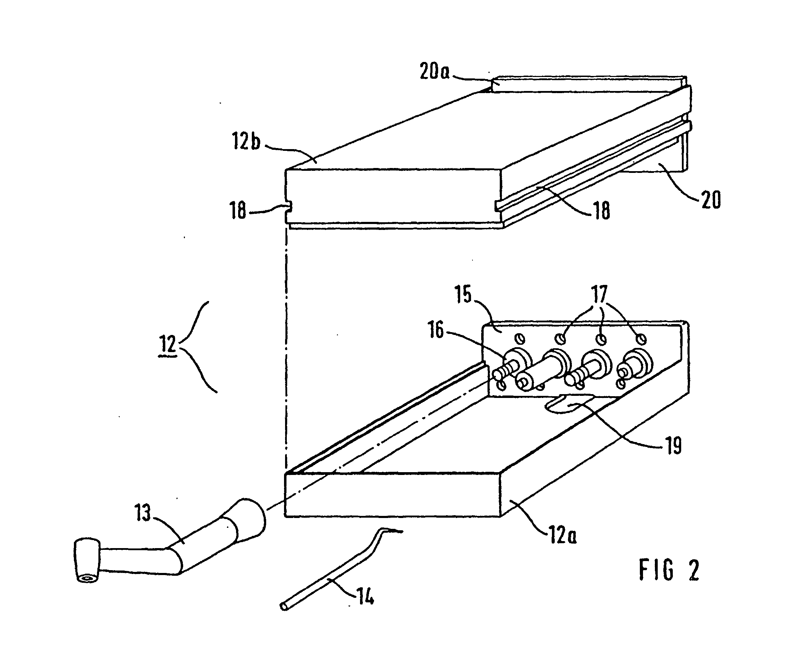

Die Figur 1 zeigt in einer schaubildlichen Darstellung eine Ausführungsform des erfindungsgemäßen Gerätes, welches hier zur hygienischen Aufbereitung diverser zahnmedizinischer Instrumente dient. Das Gerät enthält ein allgemein mit 1 bezeichnetes Gehäuse, welches frontseitig eine Kammer 2 zur Aufnahme einer später noch näher erläuterten Kassette bildet. In die Kammer 2 ragen eine Vielzahl (hier neun) von Düsenarmen 3, mit deren Hilfe, wie ebenfalls später noch näher erläutert, die Instrumente außen besprüht werden können. Beidseitig der Kammer 2 sind Führungsleisten 4 zur Führung der in Figur 2 näher dargestellten Kassette angeordnet. Die Kammer 2 ist in bekannter Weise frontseitig mittels eines Dekkels 5 druckdicht verschließbar.

Im rückwärtigen Teil des Gehäuses 1 befindet sich an der Oberseite die Einfüllöffnung 6 eines nicht dargestellten Behälters für demineralisiertes bzw. destilliertes Wasser. Seitlich daneben ist eine abdeckbare Nische zur Aufnahme eines Schmierölvorratsbehälters 7 vorgesehen. Dieser Behälter kann in bekannter Weise einen Membranverschluß aufweisen, der beim Aufsetzen auf einen entsprechend ausgebildeten Anschlußzapfen von diesem durchstochen wird, wodurch eine Entnahme des Schmieröls ermöglicht ist. Mit 8, 9 und 10 sind Zuleitungen für elektrische Energie, Druckwasser sowie Druckluft bezeichnet. Mit 11 ist ein leicht entnehmbares und damit austauschbares Sterilfilter bezeichnet, welches im Leitungskanal der Druckluftzuleitung einsetzbar ist. Damit kann sichergestellt werden, daß weitgehend keimfreie Luft für die nachfolgend näher erläuterten Verfahrensschritte zur Verfügung steht.

Die Figur 2 zeigt in einer schaubildlichen Explosionsdarstellung eine in die Kammer 2 einsetzbare Kassette 12 zur Aufnahme mehrerer Instrumente 13 und 14, die hygienisch aufbereitet werden sollen. Das in der Figur mit 13 bezeichnete Instrument ist ein Hand- und Winkelstück, welches im Innern diverse Antriebsteile und Lager sowie Medienkanäle aufweist. Das Instrument 14 dagegen ist ein Handinstrument, welches keine bewegten Teile und auch keine Medienkanäle aufweist.

Die Kassette 12 besteht aus einem Basis- oder Unterteil 12a und einem abnehmbar daran gehalterten Kassettenoberteil 12b. Das Kassettenunterteil 12a ist wannenförmig ausgebildet und enthält an einer erhöhten Rückwand 15 vier verschiedene Adapter zur Aufnahme unterschiedlich gestalteter Instrumente. Im dargestellten Ausführungsbeispiel ist der Adapter 16 auf das aufzunehmende Instrument 13 abgestimmt. Die vier Adapter sind für Instrumente vorgesehen, die bewegte Innenteile und Medienkanäle aufweisen, wie z.B. Hand- und Winkelstücke, Turbinen-, Zahnsteinentfernungs- oder Spritzhandstücke. Instrumente, die keine bewegten Innenteile haben und auch keine Medienkanäle beinhalten, wie beispielsweise das in der Figur mit 14 bezeichnete Handinstrument, werden zur Aufbereitung mittels geeigneter Halterungen am Boden des Kassettenunterteils plaziert.

Die Rückwand 15 des Kassettenunterteils 12a enthält (neun) Bohrungen 17, durch die beim Einsetzen der Kassette 12 in die Kammer 2 die dort vorhandenen (neun) Düsenarme 3 hindurchgreifen können. Die Anordnung der Düsenarme 3 und der mit ihnen korrespondierenden Bohrungen 17 sind, wie aus der Abbildung hervorgeht, jeweils dreieckförmig um die mittig gelegenen Adapter angeordnet.

Das auf das Kassettenunterteil 12a aufsetzbare und mit diesem rastend verbindbare Oberteil 12b enthält seitliche Führungsnuten 18, die mit den bereits erwähnten Führungsleisten (Pos. 4 in Fig. 1) zusammenwirken und eine exakte Zentrierung und Führung der Kassette in der Kammer 2 gewährleisten.

Die Kassette ist an geeigneter Stelle, vorzugsweise bodenseitig und dicht benachbart der Rückwand 15 mit einer oder mehreren Ablauföffnungen 19 für den Ablauf des zugeführten Reinigungs- sowie Kondenswassers versehen.

Vorteilhafterweise ist (sind) diese Ablauföffnung(en) mit einem beim Entnehmen der Kassette selbsttätig verschließenden Teil, z.B. mit einem federbelasteten Schieber oder Deckel, versehen.

Das Kassettenoberteil 12b ist vorteilhafterweise an der Rückseite mit einer Abdeckung versehen, welche die Öffnungen 17 und die zu den Adaptern 16 führenden Anschlüsse sowie gegebenenfalls die Ablauföffnung 19 abdeckt bzw. verschließt, wenn die Kassette aus dem Gerät herausgenommen wird.

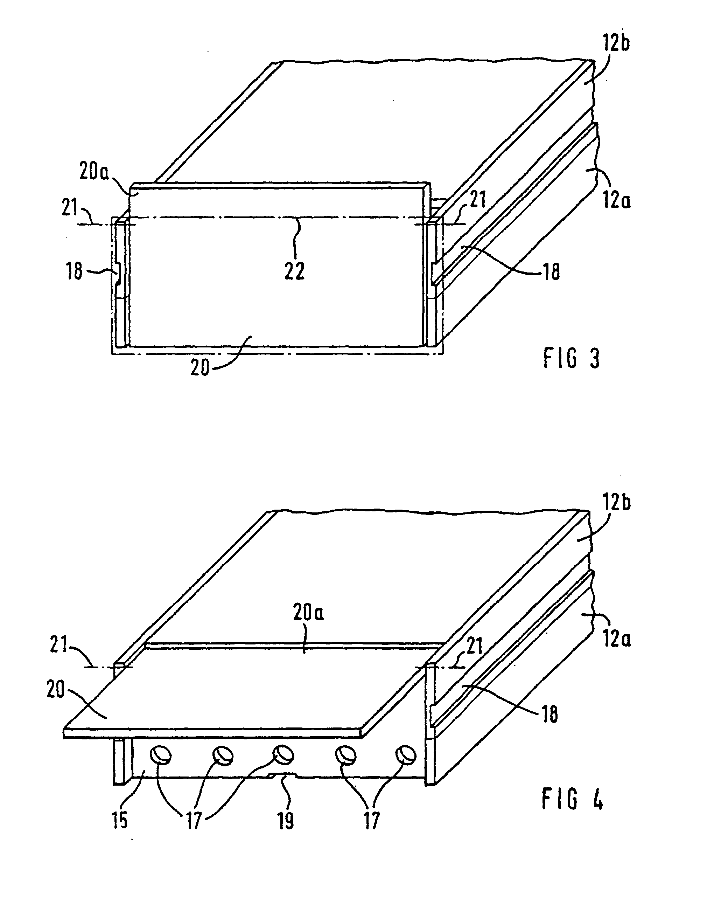

Die Figuren 3 und 4 zeigen eine Ausführungsform einer solchen Abdeckung, wobei die Figuren die Kassette von der Rückseite zeigen. Eine Abdeckplatte 20 ist um ein Lager 21 nach oben aufklappbar. Das Aufklappen geschieht automatisch beim Einschieben der Kassette 12 in die Kammer 2 des Gerätes 1. Hierzu weist die Abdeckplatte 20 einen Überstand 20a auf, der beim Einschieben der Kassette in die Kammer 2 an der mit 22 angedeuteten Oberkante der Kammer 2 anschlägt, wodurch die Klappe nach oben geöffnet wird (Fig. 4). Die dahinter befindlichen Öffnungen 17 sowie die aus der Abbildung nicht ersichtlichen Anschlüsse zu den Adaptern 16 werden sodann freigegeben. Beim Herausnehmen der Kassette klappt die Abdeckplatte 20 automatisch wieder nach unten und verschließt so die Öffnungen, so daß die in der Kassette befindlichen, hygienisch aufbereiteten Instrumente gegen Eindringen von Keimen zumindest für einen bestimmten Zeitraum geschützt sind.

Alternativ zu der aufgezeigten, selbsttätig öffnenden und schließenden Klappe kann auch ein Rolladenelement vorgesehen werden, welches mit Hilfe entsprechend vorgesehener Stellmittel eine selbsttätige Abdeckung bzw. Freihaltung der an der Rückwand 15 vorgesehenen Öffnungen bzw. Anschlüsse bewirkt.

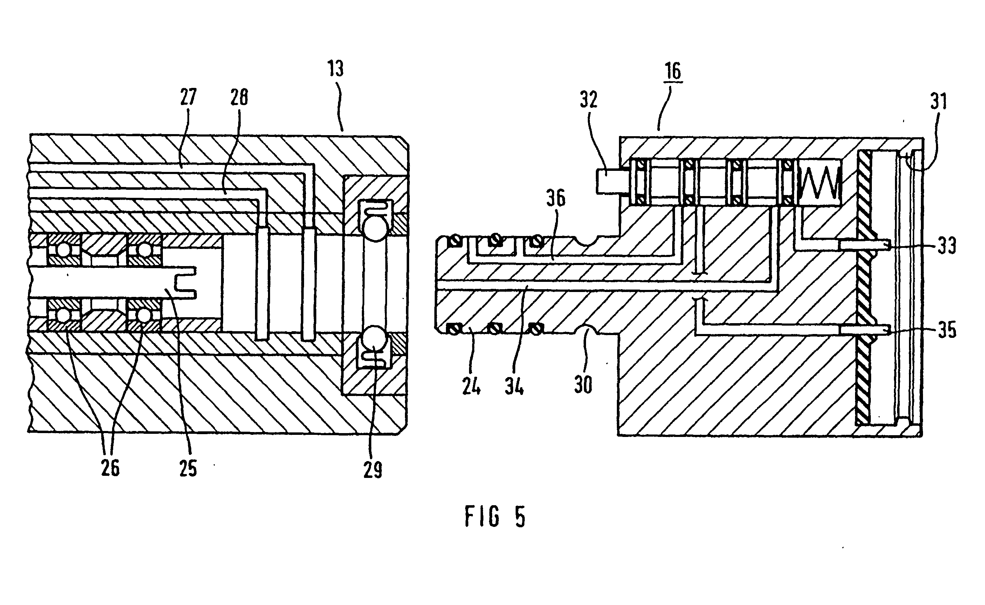

Anhand der Figur 5 wird am Beispiel des Adapters 16 der Aufbau und die Funktion der Adapter sowie das Zusammenwirken mit den daran aufsteckbaren Instrumenten erläutert.

Der Adapter 16 ist hier zum Anschließen eines in Figur 2 mit 13 bezeichneten Hand- und Winkelstückes vorgesehen, welches in seinem Inneren in Rotation versetzbare Triebwellen 25 sowie diesen zugeordnete Lager 26 enthält. Des weiteren verlaufen im Inneren eines solchen Handstückes Medienkanäle 27, 28 zur Zuführung von Kühlluft und Kühlwasser an die Präparationsstelle. Mit 29 ist eine Kugelrasteinrichtung bezeichnet, die beim Aufsetzen des Instruments 13 an einen diesem zugeordneten Antrieb mit einer entsprechenden Ringnut korrespondiert. Eine solche Ringnut ist beim Adapter 16 vorgesehen und dort mit 30 bezeichnet.

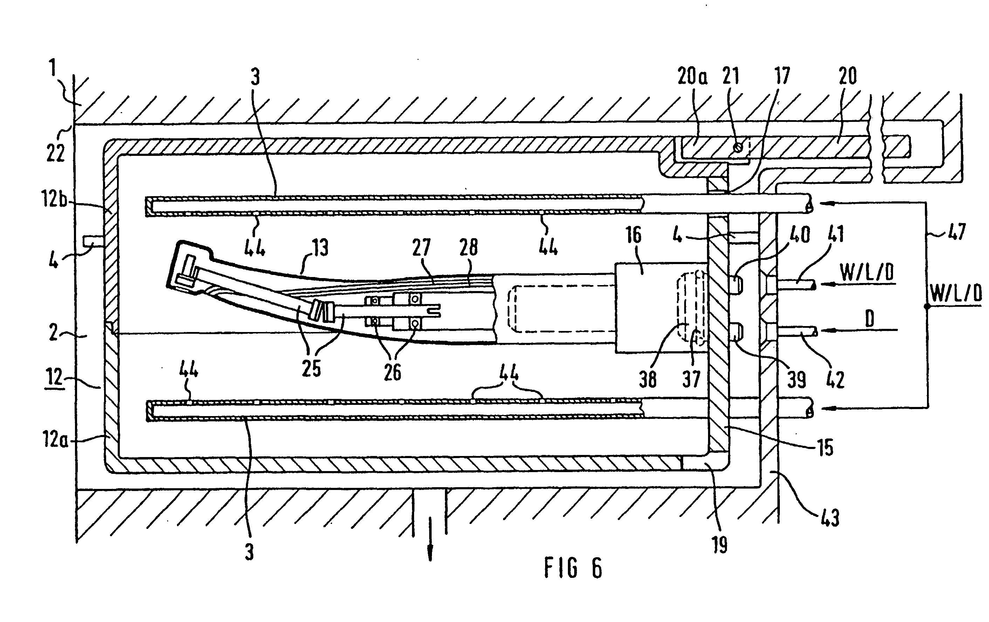

Der Adapter 16 enthält einen Anschlußzapfen 24, der baulich auf die Konstruktion des Instruments abgestimmt ist. An dem dem Instrument abgewandten Ende ist der Adapter 16 so ausgebildet, daß er an der Rückwand 15 des Kassettenunterteils 12a leicht lösbar befestigt werden kann. Hierzu enthält er eine Rastnase 31, die mit einer Ringnut 37 eines Flansches 38 (Fig. 6) an der Rückwand 15 des Kassettenunterteils 12a zusammenwirkt.

Im Adapter 16 befindet sich ein achsparallel verschiebbarer Schaltplunger 32, welcher beim Aufschieben des Instruments 13 entgegen der Kraft einer nicht näher bezeichneten Feder betätigt wird. Bei Betätigung wird der Eingangskanal 33 mit einem zentrisch gelegenen Kanal 34 verbunden. Außerdem wird die Verbindung eines weiteren Eingangskanales 35 mit einem Leitungskanal 36 hergestellt, der im aufgesetzten Zustand des Instruments über periphere Öffnungen mit den Medienleitungen 27, 28 des Instruments verbunden ist. Über den Eingangskanal 33 kann Treibluft oder Schmieröl zu den Triebwellen 25 und deren Lager 26 geleitet werden; über den Leitungskanal 35 kann Luft und Wasser gemeinsam zu den Leitungen 27 und 28 geführt werden.

Mit dem Plungerventil 32 ist sichergestellt, daß bei nicht mit einem Instrument belegtem Adapter die zugeführten Medien am Adapter nicht austreten können.

Wie bereits erwähnt, sind die Anschlußmaße bezüglich der Instrumente für alle vier in der Kassette gehalterten Adapter unterschiedlich, um so unterschiedliche Instrumente haltern zu können. Die Anschlußmaße bezüglich des Anschlusses an der Rückwand 15 der Kassette sind jedoch für alle Adapter gleich, so daß sie sehr leicht untereinander ausgetauscht bzw. gegen solche mit anderen Anschlußkonturen für andere Instrumente gewechselt werden können.

Anhand der Figur 6 wird am Beispiel des Instruments 13 und des Adapters 16 die Zuordnung der einzelnen Wasch- und Reinigungsdüsen bzw. -kanäle erläutert.

Die Figur 6 zeigt in einer stark vereinfachten Darstellung die Anordnung der Kassette 12 in der Kammer 2 in nahezu vollständig eingeschobenem Zustand. An der Rückwand 15 der Kassette 12 befinden sich, entsprechend der Anzahl der vorhandenen Adapter, Anschlußflansche 38, auf die, wie bereits erwähnt, die Adapter 16 leicht lösbar aufgesetzt werden können. Die Anschlußflansche 38 weisen Verbindungsmuffen 39, 40 auf, welche bei völlig eingeschobener Kassette eine Verbindung herstellen, einerseits zwischen Zuleitungen 41, 42 und den bereits erwähnten Eingangskanälen 33 und 35 (Fig. 5). Die um das Instrument 13 stern- bzw. dreieckförmig herum angeordneten Düsenarme 3 sind an der Gehäusewandung 43, welche die Kammer 2 rückseitig begrenzt, fest angeordnet. Sie sind als Hohlleitungen ausgebildet und weisen eine Vielzahl von Düsenaustrittsöffnungen 44 auf, die unter einem bestimmten Winkel auf die Oberfläche des Instruments 13 ausgerichtet angeordnet sind. Die Düsenbohrungen sind vorteilhafterweise längs der Düsenarme nicht nur in einer Ebene, sondern in mehreren um einen bestimmten Winkel gegeneinander versetzten Ebenen angeordnet. Der Versatz kann vorteilhafterweise zick-zack- oder wellenförmig in Längsrichtung entlang einer Bezugsebene angeordnet sein.

Wie aus Figur 1 und auch aus dem nachfolgend noch näher erläuterten hydraulisch-pneumatischen Schaltplan hervorgeht, sind zur Aufbereitung von maximal vier Instrumenten vorteilhafterweise neun Düsenarme vorgesehen. Die Anordnung ist dabei so getroffen, daß die Düsenarme jeweils in einem Winkel von 120° zueinander um ein Instrument herum angeordnet sind.

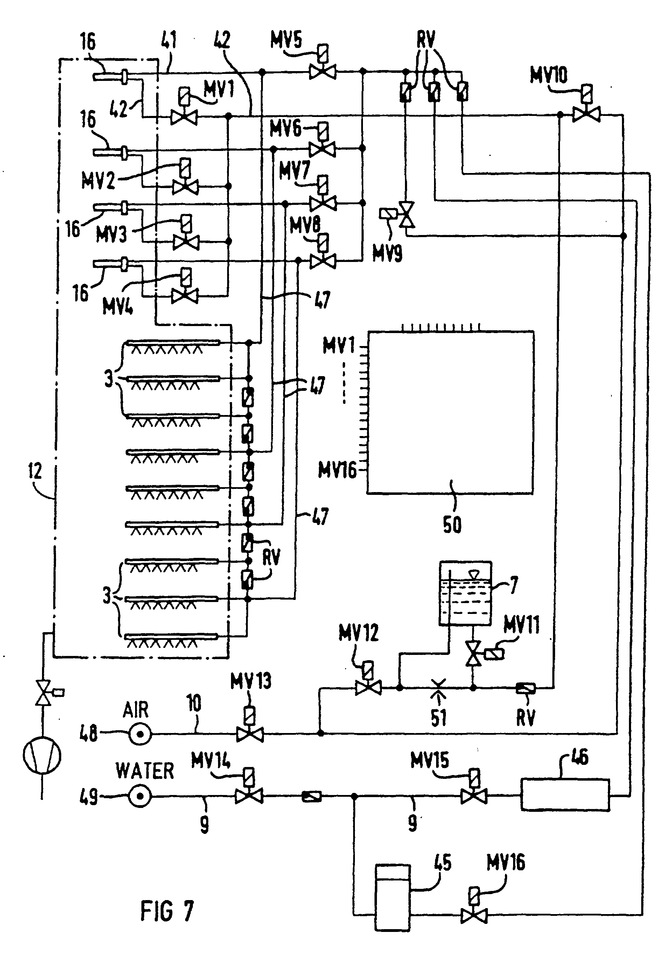

Bevor ein vorteilhaftes Reinigungsverfahren unter Einsatz der erfindungsgemäßen Vorrichtung anhand der bereits erläuterten Zeichnung und des hydraulischpneumatischen Blockschaltbildes gemäß Figur 7 näher erläutert wird, sei noch erwähnt, daß im Gerätegehäuse außer dem bereits erwähnten Ölbehälter 7 auch ein an die Druckwasserleitung 9 angeschlossener Dampferzeuger 45 sowie eine Vorwärmeinrichtung 46 für Wasser untergebracht sind.

Die Figur 7 zeigt ein hydraulisch-pneumatisches Schaltbild von der erfindungsgemäßen Einrichtung.

Mit 48 und 49 sind die Versorgungsquellen bezeichnet, mit denen einerseits Druckluft und andererseits Druckwasser in das Gerät eingespeist werden. Die eingangsseitig üblichen Filter, Ölabscheider (bei Druckluft) sowie Überdruckventile sind der Einfachheit halber nicht eingetragen.

Sämtliche Magnetventile (MV1 bis MV16) sowie die Wasserheizung 46 und der Dampferzeuger 45 werden von einem Mikroprozessor 50 aus gesteuert. Mit RV sind in die Leitungen geschaltete Rückschlagventile bezeichnet.

Die Injektion des Schmiermittels (mit Luft vermischbares Pflegeöl) aus dem Behälter 7 erfolgt hier pneumatisch über die beiden Magnetventile MV1 und MV 12 sowie eine Drosselstelle 51. Alternativ kann das Schmieröl auch mittels einer geeigneten Ölpumpe zugeführt werden. Der MP 50 steuert die Magnetventile MV1 bis MV9 und MV11 bis MV16 so, daß den Adaptern 16 und den Düsenarmen 3 die Medien Luft, Wasser und gegebenenfalls das Schmieröl bei den Verfahrensschritten der Vor- und der Nachreinigung - und gegebenenfalls beim Pflegevorgang - für jedes Instrument getrennt zugeführt werden, so daß die Instrumente zeitlich nacheinander gereinigt - und gegebenenfalls gepflegt - werden.

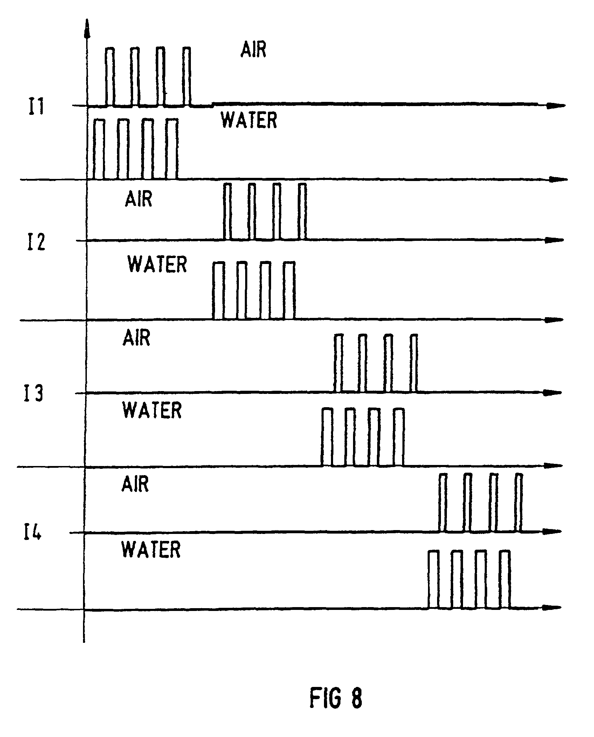

Aus Figur 8, die den zeitlichen Verlauf der Ventilanschaltung für die Medien Luft und Wasser für vier Instrumente (im Diagramm mit I1 bis I4 bezeichnet) aufzeigt, ist dieser zeitliche Versatz erkennbar. Mit dieser zeitlich aufeinanderfolgenden Zuschaltung der Medien wird bei niedrigem Wasserverbrauch eine hohe Reinigungseffizienz erzielt.

Ein vorteilhaftes Reinigungsverfahren läuft wie folgt ab:

Zunächst wird die Kassette 12 mit Instrumenten beschickt. Instrumente, die bewegbare Innenteile haben, wie Winkelstücke, Turbinen, Spritzen, od.dgl., werden auf die entsprechenden Adapter 16 (max. vier) der Kassette aufgesteckt. Die übrigen Instrumente werden an Haltevorrichtungen über dem Behälterboden plaziert. Die Kassette wird sodann durch Aufsetzen des Kassettenoberteils verschlossen und in die Kammer 2 des Gerätes eingeführt. Mit dem Einführen wird die rückwärtige Abdeckung 20 angehoben, wodurch die neun Düsenarme 3 durch die Bohrungen 17 der Kassettenrückwand hindurchgreifen können. Nachdem die Kassette in der Kammer 2 so weit eingeschoben ist, daß die Verbindung der Leitungen 41, 42 mit den Anschlüssen 39, 40 (Fig. 6) hergestellt ist, wird der Deckel 5 des Geräts geschlossen und das Gerät eingeschaltet. Nach einem Selbsttest, der etwa eine halbe Minute dauert, erfolgt zunächst eine Kaltreinigung der Instrumente, indem über die Zuleitung 9 und die Leitungen 41 und 47 den Adaptern 16 und den Düsenarmen 3 kaltes Wasser zugeführt wird. Das kalte Wasser wird vorzugsweise pulsierend zugeführt, wobei dem Wasser in den Impulspausen Druckluft mit höherem Druck (ebenfalls pulsierend) beigemischt wird (Fig. 8). Dadurch wird ein hochenergetischer Wasserstrahl erzeugt, der überraschenderweise eine sehr gründliche Reinigung der Oberfläche und auch der Innenkanäle bewirkt. Gleichzeitig wird über die Leitung 42 der Getriebekanal mit Luft beaufschlagt (das Ölzuführungsventil MV11 bleibt geschlossen), wodurch das Eindringen von Schmutz und (verschmutztem) Reinigungswasser verhindert wird. Die Impulsfrequenz, mit der dem Wasser Druckluft zugeführt wird, beträgt etwa 3 Hz.

Die Kaltreinigung ist nach etwa 2 Minuten beendet. Danach erfolgt eine Warmreinigung der Außenflächen der Instrumente und auch der Innenkanäle, ebenfalls mit einem pulsierenden Wasserstrahl, unter Beifügung von gepulster Druckluft. Dieser Vorgang läuft wie vorerwähnt ab. Nach etwa 2 Minuten Warmreinigung erfolgt eine intensive Nachreinigung und Dampfdesinfektion durch Ab- und Ausblasen der Außenflächen der Instrumente und der Medien- und Getriebekanäle im Innern der Instrumente mit Heißwasser von 60° bis 100°C. Das Wasser wird hierzu von dem als Durchlauferhitzer wirkenden Erhitzer 46 erwärmt. Dieser Vorgang dauert etwa 30 bis 45 Sekunden.

Nach dieser intensiven Nachreinigung erfolgt eine Pflege der bewegten Innenteile durch Injektion einer dosierten Menge Schmieröl aus dem Schmierölvorratsbehälter 7. Die Zudosierung kann in Abhängigkeit vom adaptierten Instrument erfolgen und beträgt im Mittel 1 Gramm pro Instrument und Injektionszyklus.

Dieser Pflegeschritt dauert etwa eine halbe Minute. Anschließend erfolgt eine Sterilisation der Instrumente, und zwar sowohl von außen als auch von innen, mittels gesättigtem Wasserdampf von vorzugsweise 134°C bei einem Druck von etwa 2 bis 2,5 bar. Der Sterilisationsvorgang beträgt zwischen 5 und 6 Minuten. Danach folgt ein Trocknen und Abkühlen der Instrumente mittels kalter Luft. Der Abkühlvorgang dauert etwa 3 bis 5 Minuten. Bei einer Gesamtzeit von etwa 15 bis 20 Minuten zur Aufbereitung der Instrumente kann danach die Kassette mit den aufbereiteten Instrumenten entnommen und im geschlossenen Zustand zur Benutzung bereitgestellt werden.

Der Verfahrensablauf, der an sich selbsttätig in der geschilderten Weise abläuft, kann durch Eingriff, z.B. durch entsprechende Programmvorwahl, die an einem Bedien- und Displayfeld 52 an der Frontseite des Gerätes abgerufen werden kann, variiert werden, z.B. indem im Anschluß an die intensive Nachreinigung und Desinfektion der Außenfläche eine Zwischentrocknung, z.B. durch Luft, durchgeführt wird. Ebenso kann nach dem Pflegen eine Unterbrechung stattfinden, um so nicht sterilisierbare Gegenstände aus der Kassette entnehmen zu können.

Zur Entfernung vorhandener Trockenluft, insbesondere aus Hohlräumen, und im besonderen aus Hohlräumen der Instrumente, kann gemäß einer vorteilhaften Variante die Kammer vor dem Sterilisieren der Instrumente evakuiert werden. Um eine optimale Entfernung der Trockenluft zu erzielen, sollte das Vakuum im Bereich zwischen 40 und 500 mbar absolut liegen. Das Vakuum kann nach dem an sich bekannten fraktionierten (mehrstufigen) Verfahren oder nach dem Vorvakuumverfahren erzeugt werden.

Mit dem geschilderten Verfahren ergeben sich extrem kurze Zeiten für eine optimale hygienische Aufbereitung der Instrumente. Durch den pulsierenden Wasserstrahl, dem stoßweise Druckluft höherer Energie zugeführt wird, ist eine besonders intensive Reinigung der Teile gewährleistet. Dadurch, daß nach der Aufbereitung die Instrumente in der Kassette verbleiben können, ist ein sicherer Transport zum Behandlungsplatz gegeben. Das gleiche gilt für den Weg nach Benutzung der Instrumente zur Aufbereitung. Die nach der Benutzung mit Keimen kontaminierten Instrumente verbleiben bis zur Wiederentnahme in der Kassette, wodurch u.a. eine Verletzungsgefahr für das Bedienpersonal ausgeschlossen ist. Bearbeitung, Lagerung und Transport erfolgen somit in einer Kassette. Nachdem die Kassette weitgehend abgeschlossen ist, ist eine sterile Lagerung der Instrumente zumindest über einen hinreichend langen Zeitraum (Stunden) gewährleistet. Dadurch, daß die Innenteile und die Medienkanäle mit Heißwasser und Dampf durchströmt werden, ergibt sich eine relativ rasche Aufheizzeit der gesamten Instrumente. Durch das pulsierende Ab- und Ausblasen der Teile mit Wasserdampf wird auch ein gewisser kalklösender Effekt in den Leitungen erzielt. Ein wesentlicher Vorteil ist, daß keinerlei Reinigungs- und Treibmittel verwendet zu werden brauchen.

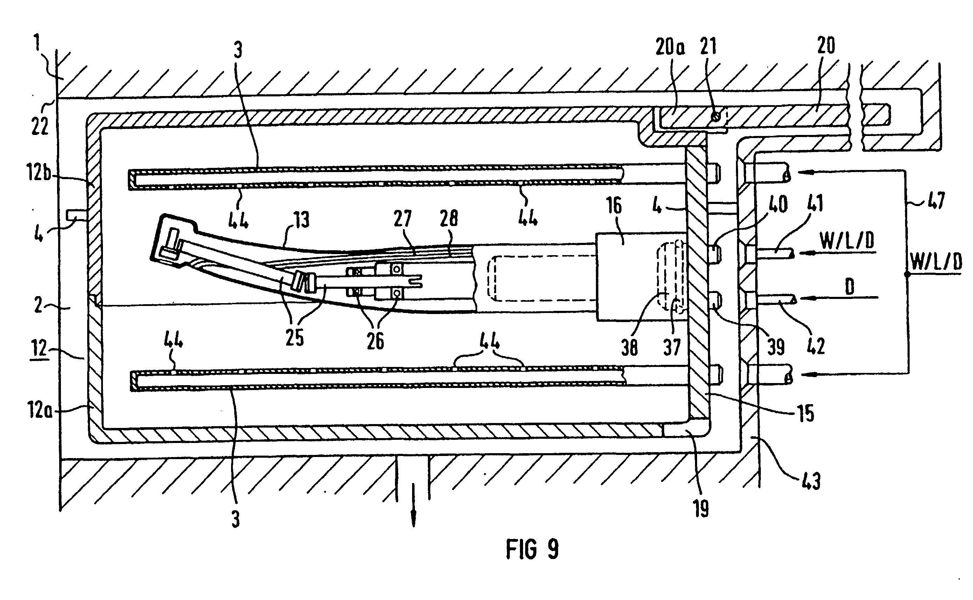

Hinsichtlich der Anordnung und Ausbildung der Düsenarme 3 sind im Rahmen der Erfindung verschiedene Modifikationen denkbar. So können beispielsweise die Düsenarme 3 auch Bestandteil der Kassette sein, wie dies im Ausführungsbeispiel nach Fig. 9 dargestellt ist. Des weiteren könnten, obgleich dies relativ aufwendig wäre, die Düsenarme um ihre Längsachse schwenkbar und gegebenenfalls auch in Achsrichtung bewegbar angeordnet sein. Die in den Fig. 3 bis 5 dargestellte Abdeckung kann alternativ auch Bestandteil des Kassettenunterteils sein oder beiden Kassettenhälften zugeordnet sein.

## Patentansprüche

1. Vorrichtung zur Intensivreinigung von ärztlichen, insbesondere zahnärztlichen Instrumenten und gegebenenfalls in deren Anwendungsbereich fallenden anderen Gegenständen (13, 14) mit Hilfe einer unter Druck zugeführten Reinigungsflüssigkeit, enthaltend eine druckdicht verschließbare Kammer (2) sowie eine die Gegestände (13, 14) aufnehmende Kassette, welche in die Kammer einlegbar ist und wobei die Kassette Adapter zur Halterung der Gegenstände (13, 14) enthält, **dadurch gekennzeichnet, daß** die Kassette eine Vielzahl von auf die gehalterten Gegenstände (13, 14) gerichteten Düsen (44) enthält, die um die Gegenstände (13, 14) herum angeordnet sind und über die die Reinigungsflüssigkeit zur Behandlung der Außenflächen der Gegenstände (13, 14) zuführbar ist.

2. Vorrichtung nach Anspruch 1, bei der zumindest für Gegenstände (13),die Medienkanäle (27, 28) und bewegte Innenteile (25) aufweisen, die Adapter (16) Mittel (24) zur Halterung der Gegenstände (13) sowie Mittel (33, 35) zur Zufuhr von fluiden und gasförmigen Medien zur Behandlung der bewegten Innenteile (25) und der Medienkanäle (27, 28) beinhalten.

3. Vorrichtung nach Anspruch 1 oder 2, bei der die Düsen (44) an Teilen der Kammer (2) angeordnet sind und die Kassette (12) mit Öffnungen für den Zu- und Ablauf der Behandlungsmedien versehen ist.

4. Vorrichtung nach Anspruch 1 oder 2, bei der die Düsen (44) an Teilen der Kassette (12) angeordnet sind und die Kassette mit Öffnungen für den Zu- und Ablauf der Behandlungsmedien versehen ist.

5. Vorrichtung nach Anspruch 3, bei der die Düsen (44) an Düsenarmen (3) angeordnet sind, die sich entlang der Kammer (2) erstrecken und am rückwärtigen Ende der Kammer (2) befestigt und so angeordnet sind, daß die Oberflächen der Gegenstände vollständig mit den Behandlungsmedien beaufschlagbar sind, wobei eine Rückwand (15) der Kassette (12) mit Öffnungen (17) versehen ist, durch die beim Einschieben der Kassette in die Kammer (2) die Düsenarme (3) hindurchgreifen.

6. Vorrichtung nach einem der Ansprüche 3 bis 5, bei der sich die Düsen (44) an Düsenarmen (3) befinden, die in einem Winkel von 120° zueinander um die Gegenstände angeordnet sind.

7. Vorrichtung nach Anspruch 6, bei der die Düsen (44) entlang der Düsenarme (3), bezogen auf eine durch die Längsachse verlaufende Bezugsebene, gegeneinander um einen kleinen Winkel versetzt angeordnet sind.

8. Vorrichtung nach Anspruch 7, bei der der Versatz so ausgerichtet ist, daß die Düsen (44) alternierend ober- und unterhalb der Bezugsebene verlaufen.

9. Vorrichtung nach einem der Ansprüche 5 bis 8, bei der die Düsenarme (3) um die Gegenstände (13) herum so angeordnet sind, daß für einen Adapter (16) die Düsen (44) von drei Düsenarmen (3) wirksam sind.

10. Vorrichtung nach Anspruch 9, bei der für zwei benachbarte Adapter (16) mindestens ein gemeinsamer Düsenarm (3) vorgesehen ist.

11. Vorrichtung nach einem der Ansprüche 1 bis 10, bei der die Adapter (16) leicht wechselbar an der Rückwand (15) der Kassette (12) gehaltert sind.

12. Vorrichtung nach Anspruch 11, bei der mindestens ein Adapter (16) zur Halterung von einem zahnärztlichen Instrument (13) vorgesehen ist, welches Leitungskanäle (27, 28) zur Zuführung der Behandlungsmedien in das Innere des Instruments aufweist.

13. Vorrichtung nach Anspruch 12, bei der der Adapter (16) ein Steuerventil (32) beinhaltet, welches die Zuführungsleitungen (33, 35) bei nicht mit einem Instrument (13) belegtem Adapter (16) verschließt.

14. Vorrichtung nach Anspruch 12, bei der mehrere Adapter (16) für unterschiedliche Instrumente (13) vorgesehen sind, wobei die Adapter bezüglich ihrer Halterung an der Gehäusewand (15) gleiche Anschlußmaße aufweisen.

15. Vorrichtung nach einem der Ansprüche 1 bis 14, bei der die Kassette (12) aus zwei durch Querteilung gebildeten und miteinander verbindbaren Gehäusehälften (12a, 12b) besteht, wobei die bodenseitige Gehäusehälfte (12a) den/die Adapter (16) trägt und die deckseitige Gehäusehälfte (12b) Führungselemente (4) zur Führung der Kassette (12) in der Kammer (3) des Gerätes aufweist.

16. Vorrichtung nach einem der Ansprüche 5 bis 10, bei der die Öffnungen (17) in der Kassette (12) für den Durchtritt der Düsenarme (3) von einem Abdeckmittel (20) verschlossen werden, wobei dem Abdeckmittel ein Steuermittel (20a) zur Freigabe der Öffnungen zugeordnet ist.

17. Vorrichtung nach Anspruch 16, bei der das Abdeckmittel eine an der Rückwand (15) der Kassette (12) schwenkbar gehalterte Platte (20) ist, die einen Überstand (20a) aufweist, der beim Einführen der Kassette in die Kammer (2) an einer Gehäusekante (22) zu Anlage kommt und dabei die Platte im Sinne einer Freigabe der Öffnungen (17) verstellt.

18. Vorrichtung nach einem der Ansprüche 1 bis 17, bei der die Kassette (12) aus einem sterilisierbaren Kunststoff besteht.

## Claims

1. A device for intensive cleaning of medical, particularly dental, instruments and optionally other objects (13, 14) used in conjunction therewith by subjection thereof to a pressurized cleaning liquid, comprising a hermetically sealable chamber (2) and a cassette which is adapted to accommodate said objects (13, 14) and which can be inserted into said chamber, which cassette contains adapters for holding said objects (13, 14), **characterized in that** said cassette contains a large number of nozzles (44) which are directed toward the mounted objects (13, 14) and disposed around said objects (13, 14), and through which the cleaning liquid can be passed for treatment of the external surfaces of said objects (13, 14).

2. A device as defined in claim 1, in which at least in the case of those objects (13) which have fluid channels (27, 28) and moving internal parts (25) said adapters (16) therefor contain means (24) for holding said objects (13) and also means (33, 35) for feeding in liquid or gaseous media for the treatment of said moving internal parts (25) and said fluid channels (27, 28).

3. A device as defined in claim 1 or claim 2, in which said nozzles (44) are disposed on parts of said chamber (2), and said cassette (12) is provided with openings for the inflow and outflow of said treatment fluids.

4. A device as defined in claim 1 or claim 2, in which said nozzles (44) are disposed on parts of said cassette (12), and said cassette is provided with openings for the inflow and outflow of said treatment fluids.

5. A device as defined in claim 3, in which said nozzles (44) are disposed in nozzle tubes (3) which extend in the longitudinal direction of said chamber (2) and are fixed to the rear wall of said chamber (2) and are arranged such that the entire surface of said objects can be subjected to the action of said treatment fluids, the rear wall (15) of said cassette (12) being provided with openings (17) for accommodating said nozzle tubes (3) when said cassette is inserted into said chamber (2).

6. A device as defined in any one of claims 3 to 5, in which said nozzles (44) are located in nozzle tubes (3) disposed around said objects at an angle of 120° to each other.

7. A device as defined in claim 6, in which said nozzles (44) are disposed along said nozzle tubes (3) out of line with each other by small angles with respect to a reference plane running through the longitudinal axis.

8. A device as defined in claim 7, in which said nozzles (44) are out of line alternately above and below said reference plane.

9. A device as defined in any one of claims 5 to 8, wherein said nozzle tubes (3) are disposed around said objects (13) such that the nozzles (44) of three nozzle tubes (3) are effective for one adapter (16).

10. A device as defined in claim 9, in which any two adjacent adapters (16) are associated with at least one common nozzle tube (3).

11. A device as defined in any one of claims 1 to 10, in which said adapters (16) are easily exchangeable mounted on the rear wall (15) of said cassette (12).

12. A device as defined in claim 11, in which at least one adapter (16) is provided for holding a dental instrument (13) which has conduits (27, 28) for feeding said treatment fluids into its interior.

13. A device as defined in claim 12, in which said adapter (16) contains a control valve (32) which closes the supply lines (33, 35) when said adapter (16) is not engaged by an instrument (13).

14. A device as defined in claim 12, in which a plurality of adapters (16) is provided for different types of instrument (13), which adapters have the same connector dimensions for mounting the same on the housing wall (15).

15. A device as defined in any one of claims 1 to 14, in which said cassette (12) consists of two interconnectable housing halves (12a, 12b) formed by transverse division of said cassette, the bottom housing half (12a) thereof carrying said adapter(s) (16) whilst the covering housing half (12b) has guide elements (4) for guiding said cassette (12) into said chamber (3) of said device.

16. A device as defined in any one of claims 5 to 10, in which said openings (17) in said cassette (12) for accommodation of said nozzle tubes (3) are closed by covering means (20), and to said covering means there is assigned a control device (20a) for opening said openings.

17. A device as defined in claim 16, in which said covering means consists of a plate (20) hingedly mounted on the rear wall (15) of said cassette (12), which plate exhibits a projecting portion (20a) which comes to bear against an edge of said housing (22) when said cassette is introduced into said chamber (2) so as to displace said plate such that said openings (17) are opened.

18. A device as defined in any one of claims 1 to 17, in which said cassette (12) is of a sterilizable plastics material.

## Revendications

1. Dispositif pour le nettoyage intensif d'instruments médicaux, notamment dentaires, et le cas échéant d'autres objets (13, 14) appartenant à leur domaine d'application à l'aide d'un liquide de nettoyage alimenté sous pression, contenant une chambre (2) obturable de manière étanche à la pression ainsi qu'une cassette recevant les objets (13, 14) laquelle peut être placée dans la chambre, la cassette contenant des adaptateurs pour fixer les objets (13, 14), **caractérisé en ce que** la cassette comprend une pluralité de buses (44) orientées vers les objets fixés (13, 14), qui sont disposées tout autour des objets (13, 14) et par le biais desquelles le liquide de nettoyage peut être acheminé pour traiter les faces extérieures des objets (13, 14).

2. Dispositif selon la revendication 1, dans lequel au moins pour les objets (13), qui présentent des conduits centraux (27, 28) et des pièces internes mobiles (25), les adaptateurs (16) comprennent des moyens (24) pour fixer les objets (13) ainsi que des moyens (33, 35) pour acheminer des milieux liquides et gazeux en vue de traiter les pièces internes mobiles (25) et les conduits centraux (27, 28).

3. Dispositif selon la revendication 1 ou 2, dans lequel les buses (44) sont disposées sur des parties de la chambre (2) et la cassette (12) est pourvue d'ouvertures pour l'apport et l'évacuation des milieux de traitement.

4. Dispositif selon la revendication 1 ou 2, dans lequel les buses (44) sont disposées sur des parties de la cassette (12) et la cassette est pourvue d'ouvertures pour l'apport et l'évacuation des milieux de traitement.

5. Dispositif selon la revendication 3, dans lequel les buses (44) sont disposées sur des bras de buses (3) qui s'étendent le long de la chambre (2) et qui sont fixés à l'extrémité arrière de la chambre (2) et disposés de telle sorte que les surfaces des objets puissent être complètement sollicitées par les milieux de traitement, une paroi arrière (15) de la cassette (12) étant pourvue d'ouvertures (17) à travers lesquelles les bras de buses (3) viennent en prise lors de l'insertion de la cassette dans la chambre (2).

6. Dispositif selon l'une quelconque des revendications 3 à 5, dans lequel les buses (44) se trouvent sur des bras de buses (3) qui sont disposés suivant un angle de 120° les uns par rapport aux autres autour des objets.

7. Dispositif selon la revendication 6, dans lequel les buses (44) sont disposées le long des bras de buses (3), par rapport à un plan de référence s'étendant à travers l'axe longitudinal, de manière décalées les unes par rapport aux autres suivant un petit angle.

8. Dispositif selon la revendication 7, dans lequel le décalage est orienté de telle sorte que les buses (44) s'étendent en alternance au-dessus et en dessous du plan de référence.

9. Dispositif selon l'une quelconque des revendications 5 à 8, dans lequel les bras de buses (3) sont disposés tout autour des objets (13) de telle sorte que les buses (44) de trois bras de buses (3) soient actives pour un adaptateur (16).

10. Dispositif selon la revendication 9, dans lequel au moins un bras de buses commun (3) est prévu pour deux adaptateurs voisins (16).

11. Dispositif selon l'une quelconque des revendications 1 à 10, dans lequel les adaptateurs (16) sont fixés de manière facilement remplaçable sur la paroi arrière (15) de la cassette (12).

12. Dispositif selon la revendication 11, dans lequel au moins un adaptateur (16) est prévu pour la fixation d'un instrument dentaire (13), lequel présente des conduits (27, 28) pour acheminer les milieux de traitement à l'intérieur de l'instrument.

13. Dispositif selon la revendication 12, dans lequel l'adaptateur (16) comprend une soupape de commande (32) qui ferme les conduites d'alimentation (33, 35) lorsque l'adaptateur (16) n'est pas pourvu d'un instrument (13).

14. Dispositif selon la revendication 12, dans lequel plusieurs adaptateurs (16) sont prévus pour des instruments différents (13), les adaptateurs présentant les mêmes dimensions de raccord par rapport à leur fixation à la paroi du boîtier (15).

15. Dispositif selon l'une quelconque des revendications 1 à 14, dans lequel la cassette (12) se compose de deux moitiés de boîtier (12a, 12b) formées par une division transversale et pouvant être connectées l'une à l'autre, la moitié de boîtier du côté du fond (12a) portant le ou les adaptateurs (16) et la moitié de boîtier (12b) du côté du plafond présentant des éléments de guidage (4) pour le guidage de la cassette (12) dans la chambre (3) de l'appareil.

16. Dispositif selon l'une quelconque des revendications 5 à 10, dans lequel les ouvertures (17) dans la cassette (12) pour le passage des bras de buses (3) sont fermées par un moyen de recouvrement (20), un moyen de commande (20a) pour la libération des ouvertures étant associé au moyen de recouvrement.

17. Dispositif selon la revendication 16, dans lequel le moyen de recouvrement est une plaque (20) maintenue à pivotement sur la paroi arrière (15) de la cassette (12), qui présente une extrémité en porte-à-faux (20a) qui vient s'appliquer lors de l'insertion de la cassette dans la chambre (2) contre une arête du boîtier (22) et déplace ainsi la plaque dans le sens d'une libération des ouvertures (17).

18. Dispositif selon l'une quelconque des revendications 1 à 17, dans lequel la cassette (12) se compose d'un plastique stérilisable.
